Europäisches Patentamt

European Patent Office

Office européen des brevets

(11) Publication number: **0 280 560 B1**

## EUROPEAN PATENT SPECIFICATION

(45) Date of publication of patent specification: **11.05.94**

(51) Int. Cl.5: **B01D 67/00**, G01N 33/546, G01N 33/569

(21) Application number: **88301655.2**

(22) Date of filing: **26.02.88**

(54) Membrane structure coated with low pI protein and methods of making and using.

(30) Priority: **27.02.87 US 19850**
**18.09.87 US 98433**

(43) Date of publication of application:
**31.08.88 Bulletin 88/35**

(45) Publication of the grant of the patent:
**11.05.94 Bulletin 94/19**

(84) Designated Contracting States:
**CH DE FR GB LI SE**

(56) References cited:
**EP-A- 0 200 381**
**EP-A- 0 257 635**
**FR-A- 2 283 903**
**US-A- 4 829 009**

**PATENT ABSTRACTS OF JAPAN, vol. 7, no. 30 (P-173)[1175], 05 February 1983&NUM;**

(73) Proprietor: **EASTMAN KODAK COMPANY (a New Jersey corporation)**
**343 State Street**
**Rochester New York 14650(US)**

(72) Inventor: **Snyder, Brian Anthony, c/o EAST-MAN KODAK Co.**
**Patent DepT.,**
**343 State Street**
**Rochester, NY 14650(US)**
Inventor: **Warren, Harold Chester, III, EAST-MAN KODAK Co.**
**Patent Dept.,**
**343 State Street**
**Rochester, NY 14650(US)**
Inventor: **Nelson, Roger Wayne, c/o EASTMAN KODAK Co.**
**Patent Dept.,**
**343 State Street**
**Rochester, NY 14650(US)**
Inventor: **Belly, Robert Troconis - EASTMAN KODAK CO**
**Patent Department - 343 State Street**
**Rochester , New York 14650(US)**

(74) Representative: **Nunney, Ronald Frederick Ad-olphe et al**
**Kodak Limited**
**Patent Department**
**Headstone Drive**
**Harrow Middlesex HA1 4TY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 280 560 B1

## Description

This invention relates to a membrane structure comprising a coated microporous membrane. It also relates to a method of making this membrane structure, and to a method of its use in agglutination methods.

Microporous membranes have been used for separation of materials for a number of applications. Such materials can be prepared from a variety of inorganic or organic materials. For instance, polyamide microporous membranes have been used for agglutination assays because of their hydrophilicity and separation properties.

U.S. Patent 4,066,512 describes membrane structures composed of microporous membranes having a coating of a water-insoluble protein such as zein or collagen and methods of making such structures. Enzymes are bound to the protein coating in order to provide a large, stable catalytic surface for biological reactions. Useful materials for preparing microporous membranes include cellulose esters and polyamides such as nylon.

While polyamide materials are useful as microporous membranes in catalytic membrane structures as described above, they present problems when used in immunoassays involving the reaction of one immunoreactive species with its receptor. Nylon membranes, in particular, are highly susceptible to nonspecific interactions with immunoreactive species, such as antibodies and antigens. That is, the immunoreactive species are likely to complex with sites on the membrane instead of with, or in the absence of, corresponding receptor molecules. Thus, when such species are immobilized on solid carrier materials (for example, polymeric beads) which are placed near the membranes in agglutination assays, unwanted interactions arise between the membranes and the carriers. These undesired interactions cause background interference and a reduction in assay sensitivity. It would be highly desirable to have membranes which do not interfere with the activity of immunoreactive species.

The water-insoluble proteins described in U.S. Patent 4,066,512 might be useful for reducing the undesirable interactions with immunoreactive species. However, those proteins require the use of organic solvents in coating procedures and therefore give rise to additional problems and concerns associated with organic solvents, such as objectionable odors, flammability and waste disposal. It would therefore be desirable to reduce or eliminate the interactions described above without the problems attendant with organic coating solvents.

EP-A-0 257 635 describes the coating of ultra filtration membranes with hydroxy-containing cellulose derivatives, polyvinylalcohol or other low molecular weight, polyfunctional amine-containing or hydroxy-containing compounds. Such materials, however, do not generally have sufficient negative charges to be useful in immunoassays.

EP-A-0 200 381 and JP 57-182170 describe the use of BSA to coat particulate reagents or membranes to reduce nonspecific interactions in immunoassays. BSA, however, is not sufficiently negatively charged to be useful to sufficiently reduce background in immunoassays.

The problems described above have been overcome with a membrane structure comprising a microporous membrane formed from a biologically inert material and having an average pore size of less than 10 micrometers,

the membrane characterized wherein it has a coating thereon comprising one or more water-soluble, negatively charged proteins, none of which has a pI greater than 5, the protein having been treated by acylation, alkylation or sulfonylation of available amine groups.

This invention also provides a method of preparing the membrane structure described above comprising

(a) providing a microporous membrane formed from a biologically inert material and having an average pore size of less than 10 micrometers, and

(b) contacting the membrane with an aqueous solution comprising one or more water-soluble, negatively charged proteins to provide a coating thereon, none of which proteins has a pI greater than 5, the protein having been treated by acylation, alkylation or sulfonylation of available amine groups, and the protein being present in the solution in an amount sufficient to provide a coating over the entire membrane without substantially diminishing the porosity of the membrane.

Further, an agglutination method for the determination of a multivalent immunoreactive species in an aqueous liquid comprises:

(a) contacting the liquid with an agglutination reagent having associated therewith receptor molecules reactive with said species so as to form an agglutinate of the reaction product of the species and the receptor molecules,

2

(b) simultaneously or subsequent to the contacting step (a), contacting the agglutinate with the membrane structure described above,

(c) separating the agglutinate from unagglutinated materials, and

(d) determining either the amount of agglutinate or unagglutinated materials.

The present invention provides membrane structures in which the nonspecific interactions of membrane with proteins or carrier materials is significantly reduced. This advantage is particularly important when the membrane structures are used in immunochemical reactions in which an immunoreactive species is caused to react with corresponding receptor molecules. As a result, the sensitivity of the immunochemical assays is greatly improved. The means for achieving these advantages is the pretreatment or coating of a microporous membrane with one or more water-soluble, negatively charged proteins, the proteins having been treated by acylation, alkylation or sulfonylation of available amine groups. None of the proteins coated thereon has a pI greater than 5.

The described proteins provide a high density of negative charge on the membrane, which charge density repels negatively charged immunoreactive species. The results are improved complexation of species with receptor, reduced background and high sensitivity in assays. Furthermore, the problems associated with organic coating solvents needed for coating water-insoluble proteins are avoided. Only the types of proteins described herein provide the unexpected results found in the practice of this invention.

The coating procedure used to prepare the membrane structures of this invention can be carried out in a matter of a few minutes, after which the membrane structure is ready for use. Alternatively, the structure can be dried and stored for an indefinite period of time without a loss in performance.

In its broadest aspects, the present invention relates to a membrane structure which can be used for a variety of purposes, including filtration, chemical and biological catalysis, diagnostic tests, separations, immunosorptions and other uses readily apparent to one skilled in the art. For example, such structures can be used for a sterilizing filtration of biological materials, filtration of hydraulic fluids and collection of bacteria or other organisms for water analysis. Alternatively, enzymes can be attached thereto and the resulting composite be used for biological catalysis as described in U.S. Patent 4,066,512, noted above.

In a preferred embodiment, the structures are useful to detect and quantify any of a wide variety of immunoreactive species (mono- or multivalent). Such species are generally antigens which have one or more sites for complexing with a corresponding receptor, that is, corresponding antibodies. Alternatively, the immunoreactive species to be detected can be an antibody which has one or more complexing sites reactive with the corresponding antigen or with an anti-antibody. Immunoreactive species which can be detected with this invention include, but are not limited to, Streptococcus A antigen, antigens from chlamydial and gonoccocal organisms, HTLV or HIV (human immunodeficiency viruses) or antibodies thereto, human chorionic gonadotropin (hCG), leutinizing hormone (LH), herpes viruses, drugs, antibiotics and other hormonal, bacterial or viral antigens and antibodies. In some instances, the species must be extracted from an organism or virus found in the biological specimen. Extraction procedures for a given species are known to one skilled in the art. Exemplary extraction procedures for Streptococcus A antigen are described below.

The microporous membrane useful in this invention is a thin porous material composed of any biologically inert material. Such materials do not interact with the immunoreactive species of interest or its receptor, and include materials, such as glass, ceramics, fibers, synthetic and natural polymers and others known in the art. Preferred materials are cellulose esters, polyamides and polyesters. The polyamides, such as nylons, are most preferred.

The membrane structure must have porosity sufficient for the intended use, whether it be filtration, assays or other likely uses. In the preferred embodiment of using the membrane structure in an agglutination assay, the porosity must be sufficient to allow fluids and unagglutinated materials to pass through but which will retain agglutinated materials. In other words, the membrane pores, after coating, must be large enough to allow passage of any reagents and unagglutinated particles, but not large enough to allow agglutinated particles to pass through. For use in an agglutination assay, the average membrane pore size is at least 5 times, and preferably from 6 to 15 times the average diameter of the agglutination reagent used therein. More particularly, the average pore size of the membrane is generally less than 10 micrometers, and preferably from 0.2 to 5 micrometers.

Useful membranes are commercially available from many sources. For example, useful polyamide materials are commercially available from Pall Corp.. One useful membrane is a nylon -66 microporous membrane manufactured and marketed by that company as BIODYNE A or ULTIPOR N-66. Useful cellulose acetate membranes are also commercially available. Useful membranes can be shaped to any desired configuration for a particular use.

As described above, the microporous membrane is coated prior to use with one or more water-soluble, negatively charged proteins. Generally, only one protein is coated thereon. However, not just any water-soluble protein will provide the unexpected results obtained by the present invention. Rather, the materials useful herein, individually, do not have a pI greater than 5 because of treatment as described below.

The term pI (or isoelectric point) is known as the pH at which there is an equal number of positive and negative charges in a molecule so that the molecule is neutral in charge. The pI of a protein can be measured using conventional materials and procedures. For example, it can be measured by isoelectric focusing using an LKB Ampholine PAG plate, pH range 3.5-9.5, and standard calibrators.

Useful water-soluble proteins include casein derivatives or other protein derivatives which are negatively charged (for example, derivatives obtained from acylation, alkylation or sulfonylation of casein, such as succinylated casein, succinylated bovine serum albumin, and succinylated collagen and other negatively charged proteins readily apparent to one skilled in the art). These materials are readily prepared by acylating, alkylating or sulfonylating a protein having available amine groups using suitable conditions. Useful acylating agents include, but are not limited to, those described in U.S. Patent 4,591,571, such as anhydrides, acyl halides and esters derived from dicarboxylic and polycarboxylic acids. Succinic anhydride is a preferred acylating agent. The preparation of succinylated casein is described below in Example 3.

Alkylating and sulfonylating agents useful in modifying the proteins for use in this invention include, but are not limited to bromoacetic acid, chloroacetic acid, fluoronitrobenzene, m-(chlorosulfonyl)benzoic acid, bromomalonic acid, bromopropionic acid and p-(chlorosulfonyl)benzoic acid.

Preferred coating materials include succinylated casein, succinylated bovine serum albumin and succinylated collagen. Succinylated casein is most preferred.

The membrane structure can be prepared by contacting a microporous membrane of desired configuration with an aqueous solution of the appropriate proteins by allowing a solution of the protein to flow through the membrane at room temperature. In some cases, heating the solution up to 40°C for several minutes may be useful. The total protein present in the solution is an amount sufficient to provide a coating over the entire membrane without substantially diminishing the porosity of the membrane. In other words, the coating should completely cover the surface of the membrane, but be thin enough so that the pores of the membrane are not blocked to an undesirable extent. At least 50% of the original porosity of the membrane prior to coating should be retained after coating. The amount of protein needed to achieve these results can be varied depending upon the membrane porosity, solution viscosity and average pore size, but it is generally an amount sufficient to coat the membrane with at least 25 $\mu$g/cm$^2$ of membrane surface. Very little of the protein is left in the coating solution after membrane coating.

The coated membrane structure can be used immediately in its wet state. However, it can also be dried for storage and later use. Any suitable drying technique can be used as long as the coating is not adversely affected.

While the membrane structures of this invention can be used in a number of ways, it is preferred that they be used to detect a multivalent immunoreactive species, such as Streptococcus A antigen. This embodiment of the invention relating to Streptococcus A antigen is presented for illustrative purposes, but it will be understood that the scope of the invention is not so limited.

A biological sample suspected of containing the Streptococcus A organism can be collected from a patient in any suitable manner. However, generally an applicator means is used to collect a biological sample by contacting the area of suspected infection with the applicator swab thereby collecting cells of Streptococcus A if they are present. Subsequently, the antigens are extracted from the organisms in a suitable manner. A preferred extraction procedure includes dipping the swab in a suitable extraction composition containing one or more reagents which singly or in combination cause release of the Streptococcus A antigen from the organism, specimen cells and other debris in the sample.

Useful extraction compositions known in the art include a mixture of nitrite salt and glacial acetic acid, as described in E.P. Publication 150,567, and enzymes derived from the bacterium Streptomyces albus as described in U.S. Patent 4,618,576. Another extraction composition is a mixture of a nitrite salt (for example, sodium nitrite or potassium nitrite) with a nonvolatile organic acid (for example, succinic or citric acid).

Extraction can be accompanied by incubation for a short period of time if desired. Centrifugation can also be used to remove extraneous material. After extraction, the medium containing the extracted antigen can be neutralized if necessary to bring the medium pH to that appropriate for antigen-antibody reaction. Such optional steps are noted, for example, by Slifkin et al, J. Clin. Microbiol. 15(1), pp. 187-189, 1982.

The presence of a multivalent immunoreactive species, for example, Streptococcus A antigen, is detected by an agglutination reagent which comprises water-insoluble particles having receptor molecules (for example, antibodies to Streptococcus A antigen) reactive with the species bound in a suitable manner to the surface of the particles. Reaction (or binding) between immunoreactive species and receptor then results in a linking together of the particles so that they form large agglutinates.

The amount of species can be detected by either measuring the amount of agglutinate or the amount of unagglutinated materials. The detection can be carried out in any suitable manner readily apparent to one skilled in the art, for example, by visually observing the amount of agglutinate. Preferably, tracer molecules are used in association with particles, as described in detail below.

Suitable particles useful in the indicator reagent can be natural or synthetic particles which are water-insoluble and capable of having a suitable number of tracer molecules associated therewith in some manner. Examples of useful particles include ferritin crystals, agarose particles, glass beads, polymeric particles, such as latex particles, and others known in the art. The following references describe representative useful particles: U.S. Patents 3,700,609, 3,853,987, 4,108,972, 4,258,001, 4,401,765, 4,419,453, 4,459,361, 4,478,946 and 4,591,571. The particles useful in an agglutination assay are generally quite small, that is less than 2 micrometers in diameter. Preferably, they have an average diameter of from 0.1 to 1 micrometer.

Particularly useful particles are polymeric latex particles, and more preferably they are what are known in the art as core-shell polymeric latex particles. A wide variety of monomers can be used in the preparation of such particles as long as the particles are water-insoluble. A worker skilled in the polymer chemistry art would be able to design and prepare suitable latex particles. Preferred core-shell polymeric latex particles used in the practice of this invention are described in Example 2 below. These particles have a core composed of homo- or copolymers of styrene, and a shell composed of homo- or copolymers of chloromethylstyrene.

The particles useful in the practice of this invention preferably have sufficient tracer molecules associated therewith in order to allow quantitative determination of the species from the amount of tracer seen in either the agglutinate or in the unagglutinated materials. The tracer molecules can be suitably attached to the outer surface of the particles, or preferably, distributed within the particles. Any tracer material which allows detection of the agglutinate can be used. If ferritin crystals are used as the particles, the tracer molecules are molecules of iron inherently in those crystals. Other natural or synthetic particles can have, as tracers: radioisotopes, colorimetric dyes, fluorescent compounds, chemiluminescent compounds, phosphorescent compounds and other detectable materials known in the art. Preferably, the tracer is a radioisotope, colorimetric dye or fluorescent compound [for example, a fluorescent dye or rare earth chelate, as described for example, in U.S. Patent 4,259,313. Most preferably, the tracer is a colorimetric dye. A worker skilled in the art would be able to combine an appropriate tracer with the particular particle used.

The tracer can be distributed within the particles in any suitable manner. For example, the tracer can be uniformly distributed therein as shown for example in U.S. Patent 3,853,987. Preferably, the tracer molecules are located in a restricted area of the particles, for example, near the surface or predominantly in the interior thereof. In the preferred core-shell particles, the tracer can be in either the core or shell, but most preferably, it is substantially in the core of the particles. In other words, very little (for example, less than 5% by weight) of the dye is in the shell portion of the particles.

Receptor molecules (for example, antibodies) reactive to the immunoreactive species to be detected (such as Streptococcus A antigen) are bound to the outer surfaces of the particles in a suitable manner, for example by adsorption or covalent attachment. Attachment can be achieved using known techniques, as described for example in the references cited above. Covalent attachment is preferred. When the receptor molecules are antibodies, either monoclonal or polyclonal antibodies can be used. Antibodies (whole or fragments thereof) can be obtained commercially or prepared using known techniques.

Simultaneously or subsequent to contact of extracted immunoreactive species with receptor molecules to form the agglutinate, the agglutinate is also contacted with the water-insoluble membrane structure of this invention. In one embodiment, the agglutinate can be formed in a separate container and then brought into contact with the membrane structure. Alternatively and preferably, the agglutinate is formed in the presence of the membrane structure, for example as mounted in a test device in which the assay is carried out.

A suitable incubation period can be used to optimize agglutination, if desired, before or during contact with the membrane. After that period, unagglutinated residual materials are washed through the membrane while leaving the agglutinate thereon. Any suitable wash fluid can be used in this step, but preferably the wash solution has a pH of from 5 to 10 and contains an ionic compound, such as a salt.

Once the unagglutinated residual materials have been washed through the membrane, the amount of immunoreactive species in either the agglutinate or unagglutinated materials can generally be determined with the unaided eye if the tracer is a readily visible colorimetric dye. Otherwise, standard colorimetric detection equipment can be used. Other types of tracers, for example, radioisotopes, fluorescent dyes, phosphorescent dyes, and the like, require suitable detection equipment.

While the present invention is not so limited, an assay for an immunoreactive species can be carried out using a suitable test device which comprises the membrane structure of the present invention. Such a device can have one or more wells into which extracted antigen is deposited for reaction with the agglutination reagent. This reagent can be added to the device during the assay, or incorporated therein at the time of manufacture. Once the agglutinate is formed, the unagglutinated materials can be washed through the membrane into a separate compartment below the membrane.

Succinylated casein was prepared by the procedure described in Example 1 below.

Succinylated bovine serum albumin was prepared by adding bovine serum albumin (200 mg) to 0.5 molar sodium phosphate buffer (10 ml, pH 8.5). Succinic anhydride (200 mg) was then added, and the resulting mixture was stirred at 25°C for three hours. The mixture was dialyzed against distilled water using SPECTRAPOR 2 dialysis tubing (Spectrum Medical Industries, Inc., Los Angeles, California). There was obtained 12 ml of product containing 1.6% solids. Sodium azide (0.01%) was added as a preservative.

Succinylated collagen was prepared by a method similar to that described for preparing succinylated bovine serum albumin to obtain 20 ml of product (1% solids).

Example 1: Membrane Structure Prepared with Succinylated Casein and Its Use in an Assay for Streptococcus A Antigen

Succinylated casein was prepared in the following manner: casein (50 g) was added to 0.5 molar sodium phosphate, dibasic (2.5 liters, pH 8.5). The resulting mixture was heated to 40°C with stirring to complete dissolution. After the heat was removed, succinic anhydride (50 g) was added to the mixture, followed by incubation under continuous stirring for three hours at ambient temperature. The resulting solution was diafiltered through a 1000 molecular weight cutoff membrane (available as (OK-PS). The initial resistivity of the effluent was 17.5 milliohms. After about 8 turnovers, the resistivity had dropped to 1.7 milliohms. The solution was then collected and diluted with deionized water to 2% solids.

A membrane structure was prepared by coating a nylon-66 microporous membrane with a succinylated casein solution (50 μl) containing 2% solids.

Antibodies to Streptococcus A antigen were covalently immobilized on core-shell polymeric particles composed of poly(styrene-co-2-acetoacetoxyethyl methacrylate)(70:30) in the core and poly(m,p-chloromethylstyrene) in the shell to provide an agglutination reagent. The average diameter of the particles was about 0.45 micrometer. These particles contained a red dye (Oil Red EGN) in the core prepared according to the teachings of Belgian Patent 843,647.

A solution of succinic anhydride (10 mg/ml dimethyl sulfoxide) was added to a suspension of the agglutination reagent described above at a weight ratio of 1 part anhydride to 1 part total protein. The resulting suspension was mixed for four hours at 25°C, centrifuged for 5 minutes at 7000 rpm and the resulting pellet was resuspended in 0.1 molar glycine buffer (pH 8.5) to a concentration of 0.3% solids.

The agglutination reagent was then mixed with Streptococcus A antigen obtained from a biological sample and incubated for two minutes at 37°C. Background controls were obtained by incubating the reagent in an extraction solution without antigen. Samples of the agglutinate (150 μl) were then placed on a membrane structure prepared with succinylated casein, as described above, and a second structure prepared with casein.

The agglutinate on the membrane structures were then washed with 1 molar tricine (200 μl, pH 8.6) to remove unagglutinated materials.

The amount of agglutinate remaining on the membrane structures was then evaluated by measuring the surface reflectance (at 530 nm) of the dye in the agglutinate and converting to $D_T$ (transmission density) using the Williams-Clapper transform (J. Opt. Soc. Am., 43, p. 595, 1953). The background levels were determined to be 0.125 for the succinylated casein membrane structure and 0.194 for the casein membrane structure. It is apparent that the succinylated casein provides a significant reduction in background over casein. The signal level of agglutinate on the succinylated casein treated structure was 0.445, and the signal for the casein treated structure was similar to that of succinylated casein. The background signal for a nylon-66 microporous membrane which was untreated was greater than 0.500, which is comparable to the signal of the agglutinate itself.

The structure treated with succinylated casein exhibited significantly reduced background and the agglutinate signal remained strong. This structure was found to exhibit high stability for long term storage.

Example 2: Comparison Example

This example compares membrane structures of the present invention with a structure similarly prepared using zein as the coating material as described in U.S. Patent 4,066,512.

Assays for Streptococcus A were carried out as follows: a microporous membrane (5 μm BIODYNE A membrane) was incorporated into a disposable device. About 40 μl of a 1% sodium phosphate solution of each of succinylated casein, succinylated bovine serum albumin, succinylated collagen or zein was added to the device, followed by addition of 40 μl of a 1 molar sodium chloride solution. A suspension of core-shell beads composed of a core of poly(styrene-co-acetoacetoxyethyl acrylate) (85:15) and a shell of poly (m&p-chloromethylstyrene-co-methacrylic acid (99.8:0.2), which had been imbibed with 3% acetonitrile solution of Oil Red EGN dye was then added. The bead composition was in glycine buffer solution (pH 8.5) and contained 0.3% solids. Extracted Streptococcus A antigen (40 μl) was then added. This mixture was incubated for 2 minutes at 37°C, then the solution was allowed to drain through the membrane. A wash solution of 1 molar sodium chloride (90 μl) was added. The dye obtained from the agglutinated beads on the membrane was measured by surface reflectance. Reflectance readings were converted to transmission values using the Williams-Clapper transform, which values are shown in Table II below.

A similar Streptococcus A assay was also run using a membrane treated with bovine serum albumin and beads containing a fluorescent europium chelate as described in U.S. Patent 4,259,313. Results of this test showed that the bovine serum albumin coating did not reduce background.

## T A B L E   I I

| Membrane Treatment \ Strep A Antigen (mg) | $D_T$ (Background) 0 | 12.4 | 266 |
|---|---|---|---|
| succinylated casein | 0.020 | 0.035 | 0.151 |
| succinylated bovine serum albumin | 0.074 | 0.138 | 0.187 |
| succinylated collagen | 0.023 | 0.037 | 0.119 |
| zein | 0.187 | 0.244 | 0.213 |

Results of these tests show the following: bovine serum albumin and zein did not reduce background. There was an improvement obtained with succinylated bovine serum albumin, but not as much as was obtained with succinylated casein. Unsuccinylated collagen was too viscous to use because it blocked membrane pores, however, background reduction obtained with succinylated collagen was similar to that obtained with succinylated casein.

## Claims

1. A membrane structure comprising a microporous membrane formed from a biologically inert material and having an average pore size of less than 10 micrometers,
the membrane structure characterized wherein it has a coating thereon comprising one or more water-soluble, negatively charged proteins, none of which has a pI greater than 5, the protein having been treated by acylation, alkylation or sulfonylation of available amine groups.

2. A method of preparing a membrane structure comprising
(a) providing a microporous membrane formed from a biologically inert material and having an average pore size of less than 10 micrometers, and
(b) contacting the membrane with a solution comprising one or more water-soluble, negatively charged proteins to provide a coating thereon, none of which proteins has a pI greater than 5, the protein having been treated by acylation, alkylation or sulfonylation of available amine groups, and the protein being present in the solution in an amount sufficient to provide a coating over the entire membrane without substantially diminishing the porosity of the membrane.

3. The method as claimed in claim 2 wherein the negatively charged protein is coated in an amount of at least 25 $\mu$g/cm$^2$ of membrane surface.

4. An agglutination method for the determination of a multivalent immune species in an aqueous liquid comprising:
   (a) contacting the liquid with an agglutination reagent having associated therewith receptor molecules reactive with the species so as to form an agglutinate of the reaction product of the species and the receptor molecules,
   (b) simultaneously or subsequent to the contacting step (a), contacting the agglutinate with a membrane structure comprising a microporous membrane formed from a biologically inert material, having an average pore size of less than 10 micrometers and having a coating thereon comprising one or more water-soluble, negatively charged proteins, none of which has a pI greater than 5, the protein having been treated by acylation, alkylation or sulfonylation of available amine groups,
   (c) separating the agglutinate from unagglutinated materials, and
   (d) determining either the amount of agglutinate or unagglutinated materials.

5. The method as claimed in claim 4 wherein the agglutination reagent has tracer molecules associated therewith.

6. The membrane structure as claimed in claim 1 wherein the biologically inert material of the membrane structure is a polyamide.

7. The membrane structure as claimed in claim 1 wherein the membrane has an average pore size of from 0.2 to 5 micrometers.

8. The membrane structure as claimed in claim 1 wherein the water-soluble, negatively charged protein is succinylated casein, succinylated bovine serum albumin or succinylated collagen.

9. The membrane structure as claimed in claim 8 wherein the water-soluble, negatively charged protein is succinylated casein.

10. An agglutination method for the determination of Streptococcus A antigen in an aqueous liquid comprising:
    (a) contacting the liquid with an agglutination reagent having associated therewith both antibody molecules for Streptococcus A antigen and tracer molecules, so as to form an agglutinate of the reaction product of the antigen and the antibody molecules,
    (b) simultaneously or subsequent to the contacting step (a), contacting the agglutinate with a membrane structure comprising a microporous membrane formed from nylon, having an average pore size of less than 10 micrometers, and having a coating thereon of succinylated casein,
    (c) separating the agglutinate from unagglutinated materials, and
    (d) determining the tracer in either the agglutinate or unagglutinated materials.

11. A membrane structure comprising a microporous membrane formed from a biologically inert material and having an average pore size of less than 10 micrometers,
    the membrane structure characterized wherein it has a coating thereon comprising one or more water-soluble, negatively charged proteins, none of which has a pI greater than 5, the protein having been treated by acylation of available amine groups.

**Patentansprüche**

1. Membranstruktur mit einer mikroporösen Membran, hergestellt aus einem biologisch inerten Material und mit einer mittleren Porengröße von weniger als 10 Mikrometern, dadurch gekennzeichnet, daß die Membranstruktur eine Beschichtung aufweist aus einem oder mehreren wasserlöslichen, negativ geladenen Proteinen, von denen keines einen pI-Wert von größer als 5 aufweist, wobei das Protein einer Behandlung unterworfen wurde durch Acylierung, Alkylierung oder Sulfonylierung von zur Verfügung stehenden Amingruppen.

2. Verfahren zur Herstellung einer Membranstruktur, bei dem man
(a) eine mikroporöse Membran, hergestellt aus einem biologisch inerten Material mit einer mittleren Porengröße von weniger als 10 Mikrometern bereitstellt, und bei man
(b) die Membran mit einer Lösung in Kontakt bringt, die ein oder mehrere wasserlösliche negativ geladene Proteine enthält, um auf der Membran eine Beschichtung zu erzeugen, wobei keines der Proteine einen pI-Wert von größer als 5 aufweist und das Protein einer Behandlung unterworfen wurde durch Acylierung, Alkylierung oder Sulfonylierung von zur Verfügung stehenden Amingruppen, und wobei das Protein in der Lösung in einer Menge vorliegt, die ausreicht, um eine Beschichtung über der gesamten Membran zu erzeugen, ohne ins Gewicht fallende Verminderung der Porosität der Membran.

3. Verfahren nach Anspruch 2, bei dem das negativ geladene Protein in einer Menge von mindestens 25 $\mu$g/cm$^2$ der Membranoberfläche aufgetragen wurde.

4. Agglutinationsverfahren zur Bestimmung einer multivalenten Immunspezies in einer wäßrigen Flüssigkeit, bei dem man:
(a) die Flüssigkeit mit einem Agglutinations-Reagens in Kontakt bringt, dem Rezeptormoleküle assoziiert sind, die mit der Spezies zu reagieren vermögen, unter Bildung eines Agglutinates aus dem Reaktionsprodukt der Spezies und den Rezeptormolekülen, bei dem man
(b) gleichzeitig oder nachfolgend zur Kontaktierungsstufe (a) das Agglutinat mit einer Membranstruktur in Kontakt bringt, die aufweist eine mikroporöse Membran, hergestellt aus einem biologisch inerten Material mit einer mittleren Porengröße von weniger 10 Mikrometern, und die hierauf aufgetragen aufweist ein oder mehrere wasserlösliche negativ geladene Proteine, von denen keines einen pI-Wert von größer als 5 aufweist, wobei das Protein einer Behandlung durch Acylierung, Alkylierung oder Sulfonylierung von zur Verfügung stehenden Amingruppen unterworfen wurde, bei dem man
(c) das Agglutinat von nicht agglutinierten Materialien trennt und bei dem man
(d) entweder die Menge des Agglutinates oder die nicht agglutinierten Materialien bestimmt.

5. Verfahren nach Anspruch 4, bei dem das Agglutinations-Reagens Tracer-Moleküle aufweist, die mit dem Reagens assoziiert sind.

6. Membranstruktur nach Anspruch 1, in der das biologisch inerte Material der Membranstruktur ein Polyamid ist.

7. Membranstruktur nach Anspruch 1, in der die Membran eine mittlere Porengröße von 0,2 - 5 Mikrometern aufweist.

8. Membranstruktur nach Anspruch 1, in der das wasserlösliche negativ geladene Protein succinyliertes Casein, succinyliertes Rinderserumalbumin oder succinyliertes Collagen ist.

9. Membranstruktur nach Anspruch 8, in der das wasserlösliche negativ geladene Protein succinyliertes Casein ist.

10. Agglutinationsverfahren zur Bestimmung von Streptokokkus-A-Antigen in einer wäßrigen Flüssigkeit, bei dem man:
(a) die Flüssigkeit mit einem Agglutinations-Reagens in Kontakt bringt, das sowohl Antikörpermoleküle für Streptokokkus-A-Antigen als auch Tracer-Moleküle assoziiert aufweist, zur Bildung eines Agglutinates aus dem Reaktionsprodukt aus dem Antigen und den Antikörpermolekülen, bei dem man
(b) gleichzeitig oder nachfolgend an die Kontaktierungsstufe (a) das Agglutinat mit einer Membranstruktur in Kontakt bringt, die eine mikroporöse Membran aufweist, hergestellt aus Nylon mit einer mittleren Porengröße von weniger als 10 Mikrometern und die eine Beschichtung aus succinyliertem Casein aufweist, bei dem man
(c) das Agglutinat von nicht agglutinierten Materialien trennt, und bei dem man
(d) den Tracer in entweder dem Agglutinat oder in den nicht agglutinierten Materialien bestimmt.

**11.** Membranstruktur mit einer mikroporösen Membran, hergestellt aus einem biologisch inerten Material und einer mittleren Porengröße von weniger als 10 Mikrometern,
dadurch gekennzeichnet, daß die Struktur eine Beschichtung aufweist aus einem oder mehreren in Wasser löslichen negativ geladenen Proteinen, von denen keines einen pl-Wert von größer als 5 aufweist, wobei das Protein durch Acylierung von zur Verfügung stehenden Amingruppen behandelt worden ist.

## Revendications

**1.** Structure membraneuse comprenant une membrane microporeuse constituée d'un matériau biologiquement inerte et ayant une taille de pores moyenne inférieure à 10 $\mu$m,
la stucture membraneuse étant caractérisée par une couche comprenant une ou plusieurs protéines chargées négativement, solubles dans l'eau, aucune de ces protéines n'ayant un pl supérieur à 5, les groupes amines disponibles des protéines ayant été traités par acylation, alkylation ou sulfonation.

**2.** Procédé de préparation de la structure membraneuse qui consiste à :
(a) prendre une membrane microporeuse constituée d'un matériau biologiquement inerte et ayant une taille de pores moyenne inférieure à 10 $\mu$m, et
(b) mettre en contact cette membrane avec une solution comprenant une ou plusieurs protéines chargées négativement, solubles dans l'eau pour former une couche sur la membrane, aucune de ces protéines n'ayant un pl supérieur à 5, les groupes amines disponibles des protéines ayant été traités par acylation, alkylation ou sulfonation, et les protéines étant présentes dans la solution en quantité suffisante pour obtenir une couche qui recouvre totalement la membrane sans pratiquement diminuer la porosité de la membrane.

**3.** Procédé selon la revendication 2 dans lequel le titre en protéines chargées négativement de la couche est égal à 25 $\mu$g/cm$^2$ de surface de la membrane.

**4.** Procédé d'agglutination pour déterminer une espèce immunologique multivalente dans un liquide aqueux qui consiste à :
(a) mettre en contact le liquide avec un réactif d'agglutination associé à des molécules réceptrices réagissant avec l'espèce pour former un agglutinat constitué du produit de réaction de l'espèce et des molécules réceptrices,
(b) simultanément à ou après l'étape (a), mettre en contact l'agglutinat avec la structure membraneuse comprenant une membrane microporeuse constituée d'un matériau biologiquement inerte et ayant une taille de pores moyenne inférieure à 10 $\mu$m, et une couche comprenant une ou plusieurs protéines chargées négativement, solubles dans l'eau, aucune de ces protéines n'ayant un pl supérieur à 5, les groupes amines disponibles des protéines ayant été traités par acylation, alkylation ou sulfonation,
(c) séparer l'agglutinat des matériaux non-agglutinés, et
(d) doser soit la quantité d'agglutinat soit la quantité de matériaux non-agglutinés.

**5.** Procédé selon la revendication 4 dans lequel le réactif d'agglutination est associé à des molécules marquées.

**6.** Structure membraneuse selon la revendication 1 dans laquelle le matériau biologiquement inerte est un polyamide.

**7.** Structure membraneuse selon la revendication 1 dans laquelle la membrane a une taille de pores moyenne comprise entre 0,2 et 5 $\mu$m.

**8.** Structure membraneuse selon la revendication 1 dans laquelle la protéine chargée négativement, soluble dans l'eau est la caséine succinylée, l'albumine de sérum de bovin succinylée ou le collagène succinylé.

**9.** Structure membraneuse selon la revendication 8 dans laquelle la protéine chargée négativement, soluble dans l'eau est la caséine succinylée.

**10.** Procédé d'agglutination pour doser l'antigène du streptocoque A dans un liquide aqueux qui consiste à :

(a) mettre en contact le liquide avec un réactif d'agglutination associé à des molécules d'anticorps du streptocoque A et à des molécules marquées pour former un agglutinat qui est le produit de réaction de l'antigène et les molécules d'anticorps,

(b) simultanément à ou après l'étape (a), mettre en contact l'agglutinat avec la structure membraneuse comprenant une membrane microporeuse constituée de nylon ayant une taille de pores moyenne inférieure à 10 $\mu$m, et une couche de caséine succinylée,

(c) séparer l'agglutinat des matériaux non-agglutinés, et

(d) doser les molécules marquées soit dans l'agglutinat soit dans le matériaux non-agglutiné .

**11.** Structure membraneuse comprenant une membrane microporeuse constituée d'un matériau biologiquement inerte et ayant une taille de pores moyenne inférieure à 10 $\mu$m, la structure membraneuse étant caractérisée par une couche comprenant une ou plusieurs protéines chargées négativement solubles dans l'eau, aucune de ces protéines n'ayant un pI supérieur à 5, les groupes amines disponibles des protéines ayant été traités par acylation.